# EUROPEAN PATENT APPLICATION

(11) **EP 4 043 003 A1**
(43) Date of publication of application: **17.08.2022**
(21) Application number: 19929184.0
(22) Date of filing: 24.12.2019
(51) Int. Cl.: A61K 8/11, A61K 8/02, A61K 8/31, A61K 8/85, A61K 8/73, A61Q 19/08, A61Q 17/00

(54) **CORE-SHELL PCM MICROCAPSULE HAVING AUTOMATIC TEMPERATURE CONTROL FUNCTION AND COOLING COSMETIC COMPOSITION COMPRISING SAME FOR EXTERNAL APPLICATION TO SKIN**

(30) Priority: 08.10.2019 KR 20190124535
(71) Applicant: Biogenics, Inc., Daejon 34036 (KR)
(72) Inventor: SHIN, Chan Jae, Daejeon 34048 (KR); NAM, Hye Jin, Seongnam-si, Gyeonggi-do 13519 (KR); OH, Jae Suk, Daejeon 34164 (KR); LIM, Sol Bin, Daejeon 34073 (KR); SON, Ji Hyun, Daejeon 35262 (KR); PARK, Jung-Hyun, Daejeon 34019 (KR)
(74) Representative: Gerauer, Marc Philippé
(86) International application number: PCT/KR2019/018390
(87) International publication number: WO 2021/071028

(57) **Abstract**

Provided are preparation of a PCM microcapsule in the form of a core-shell having an excellent heat absorption function by using a temperature-controllable phase change material (PCM) as a core material and a biodegradable polymer material as a shell material, and a cooling cosmetic composition for external skin capable of exhibiting a prevention effect of thermal aging using the same. The core-shell PCM microcapsule having an automatic temperature control function according to the present invention is prepared by dissolving a shell material and a single or mixed PCM in a solvent to produce an oil phase polymer solution, dissolving an aqueous polymer to produce an external continuous phase, emulsifying the oil phase polymer solution and the external continuous phase, and removing the external continuous phase and the solvent and carrying out drying.

## Description

### [Technical Field]

The present invention relates to preparation of a PCM microcapsule in the form of a core-shell having an excellent heat absorption function by using a temperature-controllable phase change material (PCM) as a core material and a biodegradable polymer material as a shell material, and a cooling cosmetic composition for external skin capable of exhibiting a prevention effect of thermal aging using the same.

### [Background Art]

As external activity such as going out or leisure and the like becomes frequent due to economic growth and the implementation of a five-day work week, together with a "photoaging" issue by exposure to ultraviolet rays, recently, a skin temperature rises by heat of a heater, a Korean dry sauna, a sauna, or a kitchen including infrared rays in sunlight, and the like to advance "skin aging by heat", so that symptoms such as decreased skin elasticity, an increase in wrinkles, acceleration of photoaging, and skin pigmentation occur due to an increase of a collagen decomposition promoting enzyme in the skin. Therefore, a skin temperature rise stands out as a main root cause of aging, and even in a skin care trend of new cosmetics, a ratio of products for taking care of the root cause of skin trouble rather than taking care of phenomena such as photoaging or whitening has been increased.

Conventionally, the market was formed around products including specific functions such as wrinkle improvement and UV protection, but recently, rapid growth has been accelerating mainly in the market of complex functional cosmetics including various complex antiaging functions. In particular, since outdoor activity increases due to leisure, sports, spare time life, and hobbies, the skin is often exposed to ultraviolet rays and heat which are the main causes of aging, and thus, a cooling agent for blocking ultraviolet rays and heat and various functional products using the same are currently actively developed in the cosmetic field.

In the fashion industry, in an era where a phenomenon that casual wear and outdoor wear dominate the lifestyle is prominent due to a five-day work week, a high value-added differentiated product market using temperature control materials is formed and developed, and also, a product market which is differentiated as "well-being materials" using PCM which is a temperature controlling material to allow experience of a direct effect of cold and hot changes is being formed.

A PCM is a material having a function of absorbing an amount of heat depending on an ambient temperature change to store and discharge heat and uses latent heat as an energy source, and the higher the latent heat, the better the heat storage capacity. The PCM may be classified into organic-based, inorganic-based, and eutectic mixture-based PCMs according to the type, but in the cosmetic field where the raw materials usable on skin are limited, an organic PCM which is chemically stable and has no phase separation or a supercooling phenomenon is appropriate, and a paraffin-based PCM which has higher latent heat than other organic-based PCMs is appropriate for use.

In the case of conventionally developed cooling agents, methods of preparing a cooling cosmetic composition using menthol, peppermint, peppermint oil, camphor, herbal extract, and the like which may induce a temporary skin temperature lowering effect to provide a feeling of refreshment or a feeling of cooling have been used, but the effect and the duration thereof are very temporary and the real effect of lowering skin temperature is not great.

In order to physically apply the PCM to fabric or cosmetics, a stabilization technology of encapsulating the PCM into a microcapsule form using a wall material (shell) to prevent the PCM melted by ambient temperature from leaking in a liquid state, protecting the PCM from cleaning, dry cleaning, weather, and the like, blocking deterioration and stability decrease due to leakage in a cosmetic formulation, or the like, is required.

Since the PCM was first developed by National Aeronautics and Space Administration (NASA) for using it in the aerospace industry and a PCM microcapsule was applied to functional fiber by Outlast Technologies, the PCM was actively applied in the domestic and foreign textile industry field and is in the state of being in demand in a wide range of fields, but most the PCM materials developed so far are limited to a living material field including fiber and research for suggestion of various microcapsule wall materials and characteristic comparison depending on wall material change is insufficient.

Since, at present, 90% or more of PCM microcapsule-related technologies which are currently widely used in the industry field are encapsulated by a polymerization reaction using a surfactant and a nucleator while using polymer materials such as polymethyl methacrylate (PMMA), polystyrene (PS), melamine, and polyurethane (PU), which cause serious microplastic environmental issues together with a residual monomer problem, as a wall material (Korean Patent Registration No. 1003370250000, Korean Patent Registration No. 1007415660000, Korean Patent Registration No. 1018484130000, Korean Patent Registration No. 1005558810000, International Patent Application No. PCT/ EP2012/052383, Korean Patent Registration No. 1015745220000, and International Patent Application No. PCT/EP2010/ 059888), it is impossible to use the PCM in the cosmetic field in which materials which may be used for a human body are limited and a high level of legal regulation is required and most of the PCMs are aqueous formulations based on water, and thus, cases of development in the cosmetic fields are currently extremely insufficient.

### [Disclosure]

### [Technical Problem]

An object of the present invention is to provide a PCM microcapsule in the form of a core-shell having an excellent heat absorption function and a cooling cosmetic composition for external skin capable of exhibiting a prevention effect of aging due to heat using the same, using a temperature-controllable phase change material (PCM) as a core material and a biodegradable wall material as a shell material.

### [Technical Solution]

In one general aspect, a core-shell PCM microcapsule having an automatic temperature control function includes an inner core of a PCM and an outer shell of a biodegradable polymer.

Preferably, the PCM may be included at 5 wt% to 80 wt% with respect to a total weight of the microcapsule composition.

Preferably, as the PCM, n-octacosane, n-heptacosane, n-hexacosane, n-pentacosane, n-tetracosane, n-tricosane, n-docosane, n-heneicosane, n-eicosane, n-nonadecane, n-octadecane, n-heptadecane, hexadecane, n-pentadecane, n-tetradecane, n-tridecane, stearic acid, and derivatives or composites thereof may be used alone or in combination, and use of mixed PCMs allows melting point control.

Preferably, the PCM of a cooling cosmetic is n-octadecane and n-eicosane having a melting point of 28-35°C and these may be used alone or n-docosane having a melting point of 44°C may be mixed with n-octadecane at a constant ratio.

Preferably, as the biodegradable polymer, polycaprolactone, a polylactic acid, water-insoluble cellulose, polyhydroxybutyrate, and aliphatic unsaturated polyester compounds may be used alone or in combination.

Preferably, the core-shell PCM microcapsule may further include a functional substance such as menthol, a natural oil, a synthetic oil, an essential oil, a fragrance, a vitamin, a ceramide, and an organic UV absorber at a weight ratio of 1 to 50 with respect to the weight of the PCM, inside the capsule.

Preferably, the core-shell PCM microcapsule may have an average particle size of 0.05 µm to 50 µm.

In another general aspect, a method of preparing a core-shell PCM microcapsule having an automatic temperature control function includes: dissolving a shell material which is a biodegradable polymer and a PCM which is a core material in a solvent to produce an oil phase polymer solution, dissolving an aqueous polymer to produce an external continuous phase, emulsifying the oil phase polymer solution and the external continuous phase, and removing the external continuous phase and the solvent and carrying out drying.

In still another general aspect, a cosmetic composition for external skin includes 0.1 wt% to 30 wt% of the core-shell PCM microcapsule with respect to a total weight of the composition.

### [Advantageous Effects]

In the case in which a cosmetic composition for external skin is applied to skin, the core-shell PCM microcapsule prepared according to the present invention effectively absorbs human body heat when a skin temperature rises in summer or under a high-temperature environment due to excellent heat absorption ability and a long lasting cooling effect by a large amount of latent heat and stable phase transition cycle characteristics of the PCM microcapsule, so that a skin temperature is lowered to help protect skin from thermal aging.

In addition, since in the present invention, an environmentally friendly biodegradable material is used in the preparation of the core-shell PCM microcapsule, the present invention is free from a microplastic issue by a polymer such as PMMA, PS, PU, and melamine and environmentally safe, thereby having an effect of being usable for cosmetics in a human body.

In addition, since the present invention allows preparation of a core-shell microcapsule having various latent heat properties by adjusting a ratio between a core and a shell and preparation of a PCM microcapsule having a new melting point property when mixed with PCM, a microcapsule having a desired melting point property may be prepared in the industry field as well as the cosmetic field.

In addition, the present invention allows preparation of a cooling microcapsule to which various functions are added depending on the added functional substances in addition to a cooling function, by adding a functional substance together with a PCM.

### [Description of Drawings]

The above and other objects, features and advantages of the present invention will become apparent from the following description of preferred embodiments given in conjunction with the accompanying drawings, in which:
FIG. 1 is a schematic diagram representing preparation of the PCM microcapsule according to the present invention.
FIG. 2 is an SEM image from which the PCM microcapsule form prepared according to an exemplary embodiment of the present invention may be confirmed and a TEM image from which a core-shell structure thereof may be confirmed.
FIG. 3 is an SEM image representing change of a capsule form by an increase in a polylactic acid ratio of the PCM microcapsule prepared according to Example 1, Example 2, and Example 3 of the present invention.
FIG. 4 is a graph representing measurement results from a differential scanning calorimeter which shows a stable phase transition cycle according to a temperature change of the PCM microcapsule prepared according to Example 1 of the present invention.
FIG. 5 is an SEM image of the PCM microcapsule prepared according to Example 4 and Example 5 of the present invention.
FIG. 6 is a graph representing measurement results from a differential scanning calorimeter which shows a stable phase transition cycle according to a temperature change of the PCM microcapsule prepared according to Example 4 of the present invention.
FIG. 7 is SEM and TEM images of the PCM microcapsule prepared according to Example 6 of the present invention.
FIG. 8 is an SEM image of a citron essential oil-containing PCM microcapsule prepared using polylactic acid and ethyl cellulose as a wall material according to Example 7 of the present invention.
FIG. 9 is an SEM image of a menthol-containing PCM microcapsule prepared according to Example 8 of the present invention.
FIG. 10 is a graph representing a lowered skin temperature and duration after applying a BB cream formulation prepared according to Example 9 and Comparative Example 1 of the present invention to skin.
FIG. 11 is a graph representing a lowered skin temperature and duration after applying a sunscreen formulation prepared according to Example 10 and Comparative Example 2 of the present invention to skin.
FIG. 12 of a thermal imaging camera measuring a skin temperature change after applying a sunscreen formulation prepared according to Example 10 and Comparative Example 2 of the present invention to skin.
FIG. 13 is a graph representing a lowered skin temperature and duration after long-term storing a sunscreen formulation prepared according to Example 10 and Comparative Example 2 of the present invention in an oven at 50°C for 5 weeks and applying the formulation to skin.
FIG. 14 is a graph representing a lowered skin temperature and duration after applying a sunscreen formulation prepared according to Comparative Example 2 and Comparative Example 3 of the present invention to skin.

### [Best Mode]

Hereinafter, exemplary embodiments of the present invention will be described with reference to the accompanying drawings.

The core-shell PCM microcapsule having an automatic temperature control function of the present invention includes an inner core of a PCM and an outer shell of a biodegradable polymer material.

The method of preparing a core-shell PCM microcapsule having an automatic temperature control function is preferably a dry-in-liquid method including: dissolving a shell material which is a biodegradable polymer and a PCM in a solvent to produce an oil phase polymer solution, dissolving a large amount of a aqueous polymer to produce an external continuous phase, emulsifying the oil phase polymer solution and the external continuous phase, and removing the external continuous phase and the solvent and carrying out drying.

The core-shell PCM microcapsule preferably has an average particle size of 0.05 µm to 50 µm.

The PCM of the present invention may be preferably at 5 wt% to 90 wt% with respect to a total weight of the microcapsule composition. As the PCM, n-octacosane, n-heptacosane, n-hexacosane, n-pentacosane, n-tetracosane, n-tricosane, n-docosane, n-heneicosane, n-eicosane, n-nonadecane, n-octadecane, n-heptadecane, hexadecane, n-pentadecane, n-tetradecane, n-tridecane, and the like, and derivatives or composites thereof may be used alone or in combination, and materials which undergo phase transition at 45-70°C such as stearic acid may be also used. In particular, an optimal PCM which is effective in lowering a skin temperature in the cooling cosmetic field is n-octadecane and n-eicosane having a melting point of 28-35°C, and it is characterized that these are used alone or n-docosane having a melting point of 44°C is mixed with n-octadecane at a constant ratio to prepare and use paraffin having a melting point corresponding to a skin temperature change range.

As the shell material of the present invention, polycaprolactone, polylactic acid, water-insoluble cellulose, polyhydroxybutyrate, and aliphatic-based unsaturated polyester compounds which are biodegradable polymers may be used alone or in combination.

In addition, the core-shell PCM microcapsule of the present invention may contain a functional substance inside the capsule. Here, as the functional substance, menthol, a natural oil, a synthetic oil, an essential oil, a fragrance, a vitamin, a ceramide, an organic UV absorber, and the like may be used and may be applied at a weight ratio of 1 to 90 with respect to the weight of the PCM, but considering the cooling effect by the PCM, may be preferably applied at a weight ratio of 1 to 50 with respect to the weight of the PCM.

Meanwhile, the cosmetic composition for external skin according to the present invention is prepared by including 0.1 wt% to 30 wt% of the core-shell PCM microcapsule with respect to the total weight of the cosmetic composition.

Hereinafter, the Examples and the Comparative Examples of the present invention will be described in more detail, but the following Examples are intended to illustrate the present invention, and the scope of the present invention is not limited thereto only.

### <Preparation Example 1: preparation of microcapsule>

### <Example 1>

An oil phase polymer solution was prepared by completely dissolving 4 g of polylactic acid and 6 g of a PCM (n-octadecane) in 80 g of a dichloromethane solvent using an agitator, and an external continuous phase was prepared by dissolving 2 g of polyvinyl alcohol in 98 g of distilled water.

The thus-prepared oil phase polymer solution and external continuous phase were mixed and emulsified at 4000 rpm of a homomixer for 5 minutes, stirred at a speed of 500 rpm so that the dichloromethane solvent was completely removed, washed and filtered with distilled water, and dried in a vacuum dry oven to prepare a core-shell PCM microcapsule in the form of white powder.

### <Example 2>

A core-shell PCM microcapsule was prepared in the same manner as in Example 1, except that the PCM and the polylactic acid were added and dissolved at a ratio of 5:5 in the oil phase polymer solution.

### <Example 3>

A core-shell PCM microcapsule was prepared in the same manner as in Example 1, except that the PCM and the polylactic acid were added and dissolved at a ratio of 4:6 in the oil phase polymer solution.

### <Experimental Example 1>

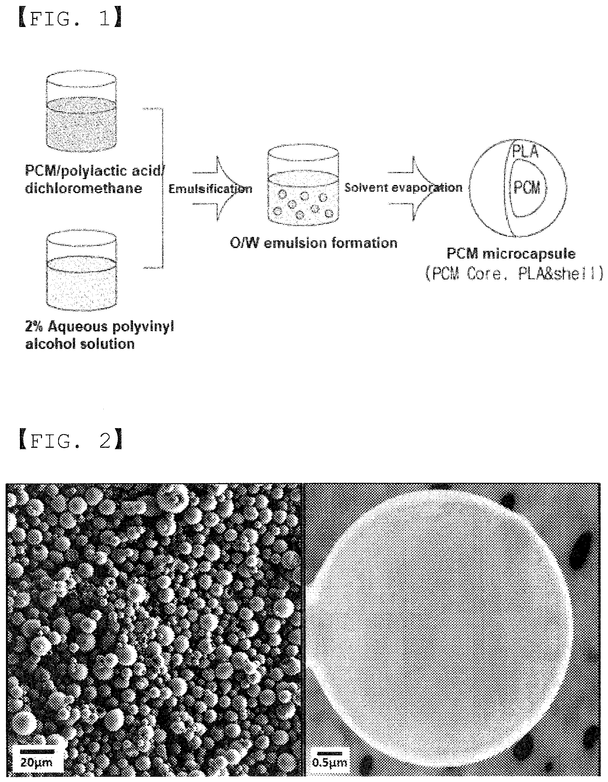

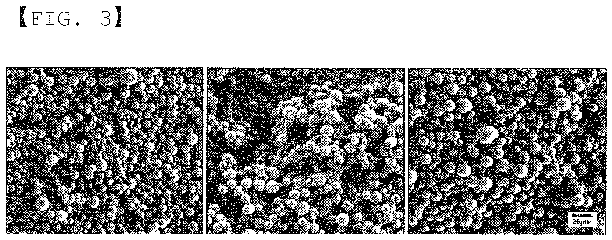

A schematic diagram representing a preparation process of the core-shell PCM microcapsule according to Example 1 is shown in FIG. 1, and an SEM image (left) in which the form of the thus-prepared core-shell PCM microcapsule may be confirmed and a TEM image (right) in which the internal structure of the core-shell may be confirmed are shown in FIG. 2. Further, SEM images representing change of a capsule form by an increase in a polylactic acid ratio of the core-shell PCM microcapsules prepared according to Example 1, Example 2, and Example 3 are shown in FIG. 3.

As shown in FIG. 2, it was confirmed by SEM analysis that the core-shell PCM microcapsules had an average size of about 8 µm and a size in a range of 2 µm to 15 µm, and it was confirmed by TEM structure analysis that a microcapsule in the form of a core-shell in which the PCM is present in the core and polylactic acid is present in a shell part was prepared, and the thickness of the polylactic acid shell was about 200 nm to 1 µm.

In addition, as shown in FIG. 3, it was confirmed that the core-shell PCM microcapsule had an increased average size with an increased ratio of the polylactic acid in the total weight of the microcapsule composition.

### <Experimental Example 2>

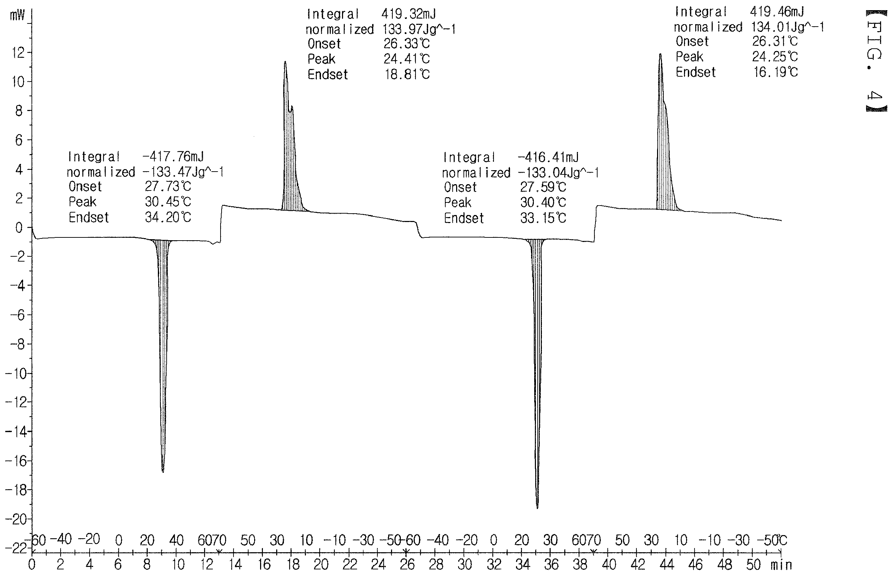

In order to confirm a stable phase transition cycle of the microcapsule according to a temperature change of the core-shell PCM microcapsule of Example 1, measurement was performed with a differential scanning calorimeter (DSC), a cycle test in which a heating temperature was changed from -60°C to 70°C → -60°C → 70°C → -60°C was performed, and the measurement results are shown in the graph of FIG. 4.

As shown in FIG. 4, it was confirmed that the core-shell PCM microcapsule was repeatedly exothermic and endothermic at the time of temperature rise and fall and had a stable phase transition cycle while preserving heat capacity even in a repeated phase transition process.

### <Experimental Example 3>

The core-shell PCM microcapsules prepared according to Examples 1, Example 2, and Example 3 of the present invention were thermally analyzed using a differential scanning calorimeter and the measurement results are shown in Table 1.

**[Table 1]**

| Classification | | Melting onset temperature (°C) | Melting temperature (°C) | Melting end temperature (°C) | Melting enthalpy (Jg⁻¹) | PCM content in capsule (%) |
|---|---|---|---|---|---|---|
| n-Octadecane | | 28.1 | 29.9 | 32.8 | 248.4 | |
| Polylactic acid (PLA) | | 167.3 | 173.4 | 176.8 | 48.4 | |
| Example 1 | PCM | 27.4 | 29.8 | 32.3 | 151.8 | 61.1 |
| | PLA | 166.3 | 171.2 | 174.7 | 19.2 | 39.7 |
| Example 2 | PCM | 27.7 | 30.5 | 34.2 | 133.5 | 53.7 |
| | PLA | 165.9 | 171.0 | 175.2 | 23.9 | 49.4 |
| Example 3 | PCM | 27.6 | 29.4 | 31.6 | 107.2 | 43.2 |
| | PLA | 166.5 | 171.3 | 175.0 | 30.7 | 63.4 |

As shown in Table 1, it was confirmed that the content of the PCM core material in the PCM microcapsule in the form of a core-shell was 61.1% in Example 1 in which a ratio of PCM:polylactic acid was applied as 6:4, which shows that the PCM was present at a very high content in the microcapsule, and a supercooling phenomenon did not occur though a nucleator which is commonly added for preventing a supercooling phenomenon was not used.

In addition, as in Example 2, when a ratio of PCM:polylactic acid was lowered to 5:5, the content of the PCM in the microcapsule was found to be 53.7%, and when a ratio of PCM:polylactic acid was lowered to 4:6 as in Example 3, the content of the PCM in the microcapsule was found to be 43.2%, and thus, it was confirmed that the content of the PCM in the microcapsule was adjustable by adjusting the ratio of PCM:polylactic acid.

### <Example 4>

A microcapsule was prepared in the same manner as in Example 1, except that as the PCM, n-octadecane and n-eicosane were mixed at a ratio of 3:7 and used in the oil phase polymer solution.

### <Example 5>

A microcapsule was prepared in the same manner as in Example 1, except that as the PCM, n-eicosane was used alone in the oil phase polymer solution.

### <Experimental Example 4>

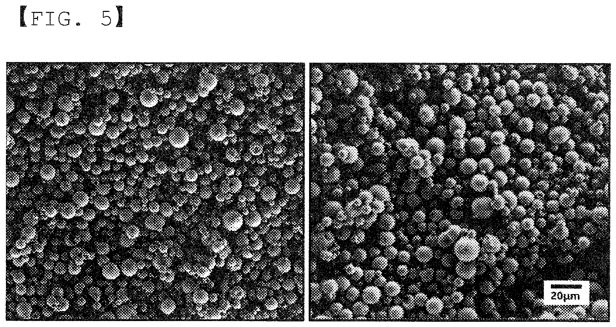

An SEM image in which the forms of the core-shell PCM microcapsules prepared according to Example 4 and Example 5 may be confirmed is shown in FIG. 5.

As shown in FIG. 5, as a result of confirming the capsule forms of the core-shell PCM microcapsules prepared according to Example 4 and Example 5 by SEM analysis, it was found that the microcapsules having similar size and form to those of Example 1 were prepared.

### <Experimental Example 5>

The core-shell PCM microcapsules prepared according to Example 4 and Example 5 of the present invention were thermally analyzed using a differential scanning calorimeter and the measurement results are shown in Table 2.

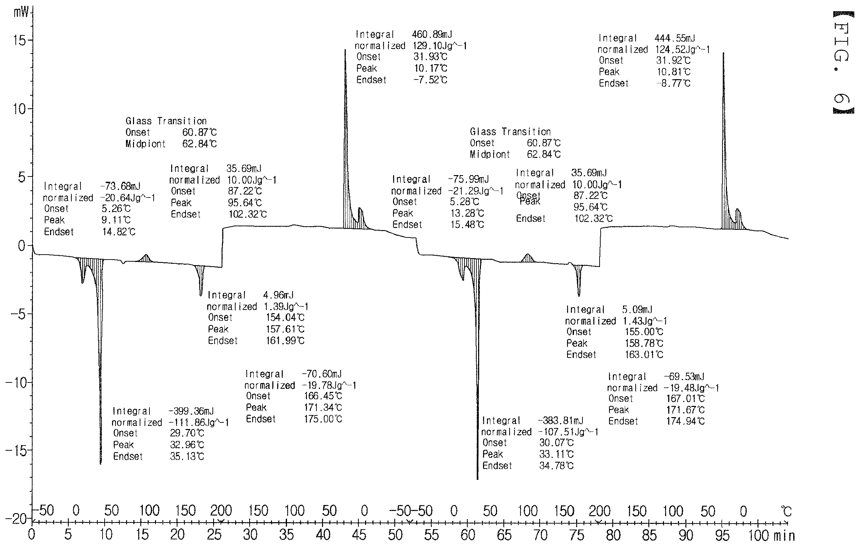

In addition, in order to confirm a stable phase transition cycle of the microcapsule according to a temperature change of the core-shell PCM microcapsule prepared according to Example 4, measurement was performed with a differential scanning calorimeter (DSC), a cycle test in which a heating temperature was changed from -60°C to 200 °C → -60°C → 200 °C → -60°C was performed, and the measurement results are shown in the graph of FIG. 6.

**[Table 2]**

| Classification | | Tₒₘ (°C) | Tₚₘ (°C) | Tₑₘ (°C) | Hₘ (Jg⁻¹) | PCM content in capsule (%) |
|---|---|---|---|---|---|---|
| n-Octadecane | | 28.1 | 29.9 | 32.8 | 248.4 | |
| n-Eicosane | | 34.9 | 37.2 | 39.8 | 223.9 | |
| Polylactic acid (PLA) | | 167.3 | 173.4 | 176.8 | 48.4 | |
| Example 4 | PCM | 29.7 | 32.9 | 35.1 | 132.5 | 57.3 |
| | PLA | 166.5 | 171.3 | 175.0 | 19.8 | 40.9 |
| Example 5 | PCM | 27.7 | 30.5 | 34.2 | 133.5 | 57.7 |
| | PLA | 166.5 | 171.8 | 175.8 | 18.9 | 39.0 |

As shown in Table 2, the contents of the PCM core material in the core-shell PCM microcapsules prepared using the mixed PCM according to Example 4 and Example 5 were 57.3% and 57.7%, and it was confirmed that the microcapsules were prepared so that the ratio of PCM:polylactic acid was close to 6:4 similarly to the case of Example 1 in which the microcapsule was prepared using a single PCM.

As shown in FIG. 6, it was confirmed that the core-shell PCM microcapsule prepared according to the Examples was repeatedly exothermic and endothermic at the time of temperature rise and fall and had a stable phase transition cycle while preserving heat capacity even in a repeated phase transition process to 200°C.

In addition, as a result of preparing the microcapsule by mixing and using two types of PCM at a constant ratio, it was confirmed that core-shell PCM microcapsules having different melting temperature properties from the used two type of PCMs without a supercooling phenomenon which may cause loss of latent heat accumulation ability were prepared.

Therefore, it was confirmed that the core-shell PCM microcapsule which allows melting temperature control and has melting temperature properties appropriate for being applied to skin in summer or cooling functional clothing may be prepared by the preparation process.

### <Example 6>

A microcapsule was prepared in the same manner as in Example 1, except that ethyl cellulose was used instead of polylactic acid as the wall material in the oil phase polymer solution.

### <Experimental Example 5>

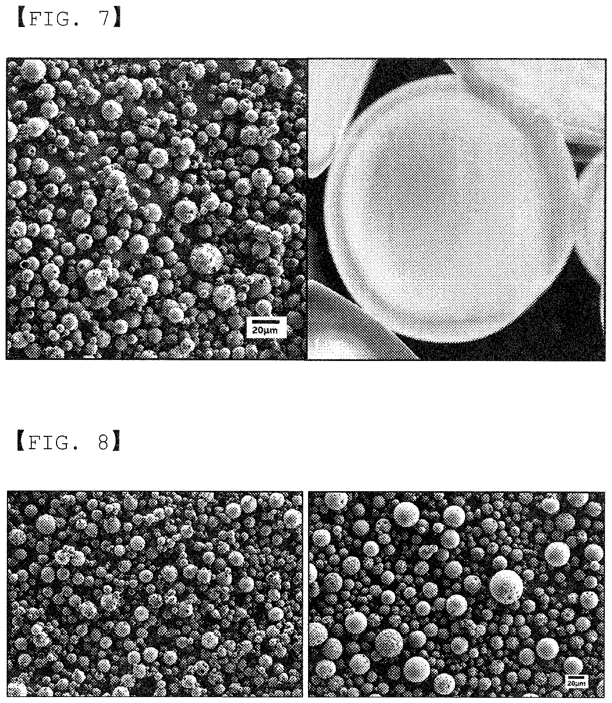

The SEM and TEM images of the PCM microcapsule prepared according to Example 6 were confirmed and are shown in FIG. 7.

As shown in FIG. 7, when the PCM microcapsule was prepared using ethyl cellulose as the wall material, the microcapsule had pores having a size of 1 µm or less on the surface, unlike the microcapsule having a smooth surface prepared by applying polylactic acid as the wall material, but it was confirmed by TEM image analysis that the core-shell PCM microcapsule having pores which were not connected to a PCM core layer and including a PCM core and an ethyl cellulose shell was prepared.

### <Example 7>

A microcapsule was prepared in the same manner as in Example 1, except that a citron essential oil as a functional substance was used at a ratio of 1:1 wt% together with the PCM as the core material and polylactic acid or ethyl cellulose was used as the shell material in the oil phase polymer solution.

### <Experimental Example 6>

The SEM image of the citron essential oil-containing PCM microcapsule prepared according to Example 7 was confirmed and is shown in FIG. 8.

As shown in FIG. 8, as a result of preparing a capsule using the PCM and a citron essential oil which is a functional substance as the core material and polylactic acid or ethyl cellulose as the shell material, it was confirmed that when using polylactic acid, a microcapsule having pores on the surface (left) was prepared, while when using ethyl cellulose, a microcapsule having a rough surface (right) was prepared. Therefore, it was confirmed that when a capsule was prepared by adjusting a ratio of PCM:fragrance oil:polylactic acid or PCM:fragrance oil:ethyl cellulose according to Example 7, microcapsules having various surface properties may be prepared, and the surface property control may be applied to release rate control of the fragrance oil depending on the size and form of the pores in the surface.

### <Example 8>

A microcapsule was prepared in the same manner as in Example 1, except that menthol which is used as a general purpose cooling agent in the cosmetic field was added at 10 wt% with respect to the wt% of the PCM together with the PCM as the core material and a ratio of menthol-containing PCM:polylactic acid = 6:4 or 3:7 was applied in the oil phase polymer solution.

### <Experimental Example 7>

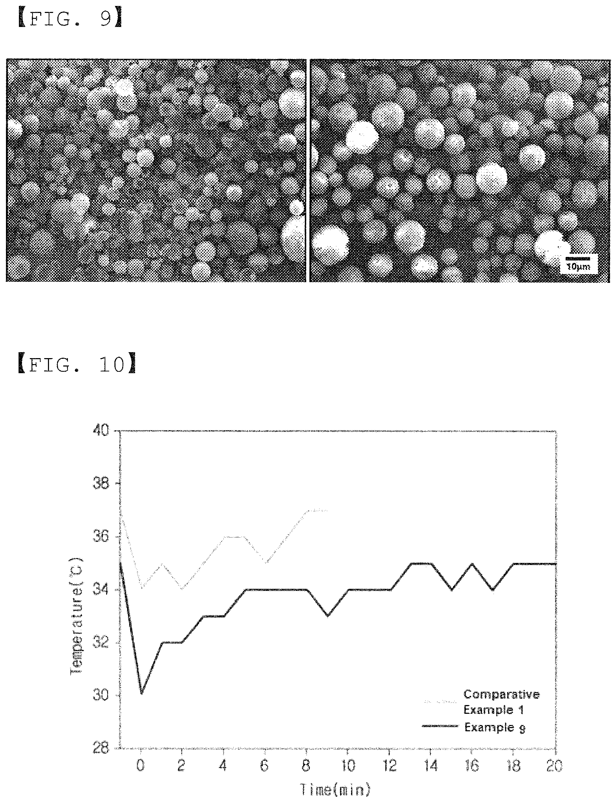

The SEM image of the menthol-containing PCM microcapsule prepared according to Example 8 was confirmed and is shown in FIG. 9.

As shown in FIG. 9, it was confirmed that the menthol-containing PCM microcapsule prepared according to Example 8 had a similar form to the PCM microcapsule of Example 1, and microcapsules having various sizes and forms may be prepared by adjusting the ratio of menthol-containing PCM:polylactic acid. In addition, it was confirmed that a cooling microcapsule having both a refreshing feeling by menthol and a cooling feeling by the PCM may be prepared.

### <Preparation Example 2> Preparation of cosmetic composition for external skin

### <Example 9: preparation of BB cream formulation>

The PCM microcapsule of Example 1 was mixed as the cooling agent and stirring was performed to prepare a BB cream formulation in the form of a cream formulation to be applied to skin. The specific components and contents of the BB cream formulation are shown in Table 3. The preparation process of each formulation proceeded in the following order:
(1) an oil phase mixture was stirred at a speed of 500-700 rpm for 5 minutes under a heating condition of 70-80°C to be completely dissolved;
(2) a powder phase was mixed with the mixture obtained in the step (1) and stirring was performed at 600-800 rpm using a homomixer so that the mixture was uniformly dispersed;
(3) an aqueous phase mixture which was completely dissolved by stirring at a stirring speed of 2800-3500 rpm for 5 minutes at 70-80°C using a homomixer was mixed with the mixture in which the powder phase was dispersed obtained in the step (2) and stirring was performed;
(4) the mixture obtained in the step (3) was cooled to 45°C, mixed with a fragrance, stirred at 2800-3500 rpm for 5 minutes, and cooled to 30°C; and
(5) the PCM microcapsule was added portionwise to the mixture obtained in the step (4) and dispersed to prepare the liquid phase cosmetic composition.

**[Table 3]**

| Components | Example (unit, wt%) | Comparative Example (unit, wt%) |
|---|---|---|
| Cyclopentasiloxane, cyclohexylsiloxane | 15.00 | 15.00 |
| Neopentylglycol diethylhexanoate | 4.00 | 4.00 |
| Butyleneglycol dicaprylate/dicaprate | 4.00 | 4.00 |
| Pentyl trimethicone | 8.00 | 8.00 |
| Ethylhexyl methoxycinnamate | 4.00 | 4.00 |
| PEG-10 dimethicone | 3.00 | 3.00 |
| Cetyl PEG/PPG-10/1 dimethicone | 1.00 | 1.00 |
| Diisostearyl maleate | 0.50 | 0.50 |
| Tocopheryl acetate | 1.00 | 1.00 |
| Disteadimonium hectorite, propylene carbonate, cyclopentasiloxane | 3.00 | 3.00 |
| Titanium dioxide, disodiumstearoyl glutamate, aluminum hydroxide | 12.68 | 12.68 |
| Nylon-12 | 1.56 | 1.56 |
| Iron oxide, triethoxycaprylsilane | 1.35 | 1.35 |
| Water | 18.88 | 18.88 |
| 1,3-BG | 10.00 | 10.00 |
| 1,2-hexanediol | 0.50 | 0.50 |
| Glycerin | 6.00 | 6.00 |
| Fragrance | Appropriate amount | Appropriate amount |
| PCM microcapsule | 3.00 | |

### <Comparative Example 1>

A BB cream formulation was prepared in the same manner as in Example 9, except that the PCM microcapsule was not contained in the BB cream formulation as the cooling agent.

### <Example 10: preparation of sunscreen formulation>

The PCM microcapsule of Example 1 was mixed as the cooling agent and stirring was performed to prepare a sunscreen formulation in the form of a cream formulation to be applied to skin. The specific components and contents of the sunscreen formulation are shown in Table 4. The preparation process of each formulation proceeded in the following order:

**[Table 4]**

| Components | Example (unit, wt%) | Comparative Example (unit, wt%) |
|---|---|---|
| Ethylhexyl methoxycinnamate | 7.00 | 7.00 |
| Butyleneglycol dicaprylate/dicaprate | 4.00 | 4.00 |
| C12-15 alkyl benzoate | 3.00 | 3.00 |
| Bis-ethylhexyloxyphenol methoxyphenyl triazine | 3.00 | 3.00 |
| Diethylaminohydroxylbenzoyl hexyl benzoate | 2.50 | 2.50 |
| Cetearyl alcohol, cetearyl glucoside | 3.00 | 3.00 |
| Cetearyl alcohol | 1.00 | 1.00 |
| Polysorbate 60 | 1.00 | 1.00 |
| Ethylhexyl triazone | 0.50 | 0.50 |
| Tocopheryl acetate | 0.10 | 0.10 |
| Sorbitan isostearate | 0.50 | 0.50 |
| Water | 49.82 | 49.82 |
| Dipropylene glycol | 6.00 | 6.00 |
| 1,2-hexanediol | 1.50 | 1.50 |
| Allantoin | 0.10 | 0.10 |
| Butyleneglycol | 1.00 | 1.00 |
| Glycerin | 0.50 | 0.50 |
| Titanium dioxide | 6.00 | 6.00 |
| PCM microcapsule | 3.00 | |

(1) an oil phase mixture was stirred at a speed of 500-700 rpm for 5 minutes under a heating condition of 70-80°C to be completely dissolved;
(2) an aqueous phase mixture was stirred at a speed of 500-700 rpm for 5 minutes under a heating condition of 70-80°C to be completely dissolved;
(3) the mixture obtained in the step (1) was mixed with the mixture obtained in the step (2) at 75°C slowly at a stirring speed of 3000-3500 rpm and stirring was performed for 3 minutes;
(4) a C-phase mixture was added to the mixture obtained in the step (3) and stirring was performed at 75°C at a stirring speed of 3000-3500 rpm for 3 minutes;
(5) a raw material D was added to the mixture of the step (4) and stirring was performed at 75°C at a stirring speed of 3000-3500 rpm for 3 minutes; and
(6) the PCM microcapsule was added portionwise to the mixture obtained in the step (5) and dispersed to prepare the liquid phase cosmetic composition.

### <Comparative Example 2>

A sunscreen formulation was prepared in the same manner as in Example 10, except that the PCM microcapsule was not contained in the sunscreen formulation as the cooling agent.

### <Comparative Example 3>

A sunscreen formulation was prepared in the same manner as in Example 10, except that menthol which is a commonly used cooling component was used as the cooling agent instead of the PCM microcapsule in the sunscreen formulation.

### <Experimental Example 8: skin temperature lowering test when applied to skin>

In order to measure the skin cooling functionality of the cosmetic composition for external skin prepared according to Example 9, Example 10, Comparative Example 1, Comparative Example 2, and Comparative Example 3, the cooling formulation was applied to skin after waiting for 20 minutes under an isothermal-isohumidity environment of 25°C and 50%, a skin temperature decrease and duration were measured, and a skin temperature change before and after applying the cooling formulation to skin was measured using an infrared thermal imaging camera (SEEK THERMAL, USA).

### <Experimental Example 8-1>

Each of the BB cream formulation containing the PCM microcapsule prepared according to Example 9 and the BB cream formulation containing no PCM microcapsule of Comparative Example 1 was applied to a forearm area, skin temperature lowering and duration were measured, and the results are shown in the graph of FIG. 10.

As shown in FIG. 10, after applying the BB cream formulation containing the PCM microcapsule of Example 9, the skin temperature was lowered by 5°C and the temperature reached the same temperature as the skin after 18 minutes. However, after applying the BB cream formulation containing no PCM microcapsule of Comparative Example 1, it was confirmed that the skin temperature was lowered by 3°C immediately after the application and the temperature reached the same temperature as the skin after 8 minutes.

As such, it was confirmed that when the PCM microcapsule prepared in the Example of the present invention was applied to the BB cream formulation, the formulation may have an excellent skin cooling function and durability.

### <Experimental Example 8-2>

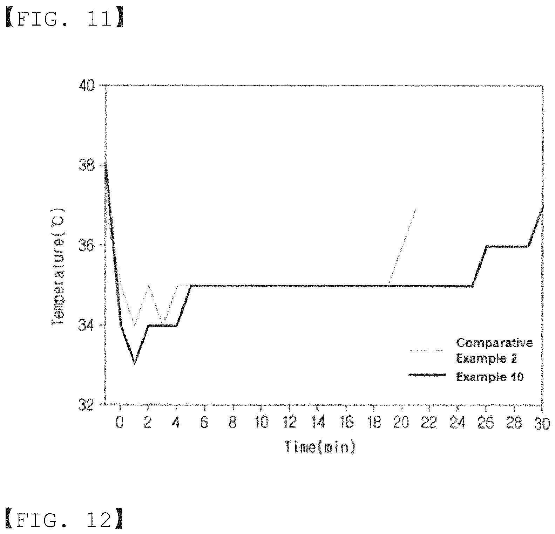

Each of the sunscreen formulation containing the PCM microcapsule prepared according to Example 10 and the sunscreen formulation containing no PCM microcapsule of Comparative Example 2 was applied to a forearm area, skin temperature lowering and duration were measured, and the results are shown in the graph of FIG. 11.

In addition, a skin temperature change before and after application to a forearm area was measured with a thermal imaging camera and is shown in FIG. 12 (photograph above: Example 10, photograph below: Comparative Example 2).

As shown in FIG. 11, after applying the sunscreen formulation containing the PCM microcapsule of Example 10, the skin temperature was lowered by 5°C and the temperature reached the same temperature as the skin after 30 minutes. However, after applying the sunscreen formulation containing no PCM microcapsule of Comparative Example 2, it was confirmed that the skin temperature was lowered by 3°C and the temperature reached the same temperature as the skin after 20 minutes.

As such, it was confirmed that when the PCM microcapsule prepared in the Example of the present invention was applied to the sunscreen formulation, the formulation may have an excellent skin cooling function and durability.

### <Experimental Example 8-3>

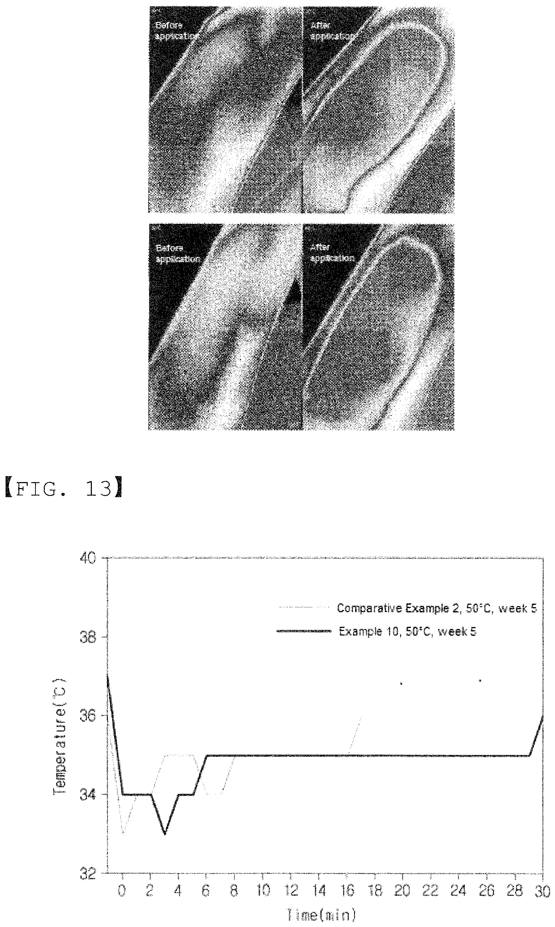

Each of the sunscreen formulation containing the PCM microcapsule prepared according to Example 10 and the sunscreen formulation containing no PCM microcapsule of Comparative Example 2 was long-term stored in an oven at 50°C for 5 weeks and then formulation stability was observed, and after applying the formulation to a forearm area for confirming the cooling functionality, skin temperature lowering and duration were measured and the results are shown in the graph of FIG. 13.

As shown in FIG. 13, when the sunscreen formulation was long-term stored in an oven at 50°C for 5 weeks, after applying the sunscreen formulation containing no PCM microcapsule of Comparative Example 2, the skin temperature was lowered by 3°C and the temperature reached the same temperature as the skin after 17 minutes; however, after applying the sunscreen formulation containing the PCM microcapsule of Example 10, the skin temperature was lowered by 4°C and the temperature maintained a difference of 1°C from the skin temperature even after 30 minutes. Thus, the long-term stability at high temperature of the cooling formulation containing the PCM microcapsule was confirmed, and it was also confirmed that the sunscreen formulation also maintained stability without abnormalities such as discoloration, changed odor, and formulation separation.

### <Experimental Example 8-4>

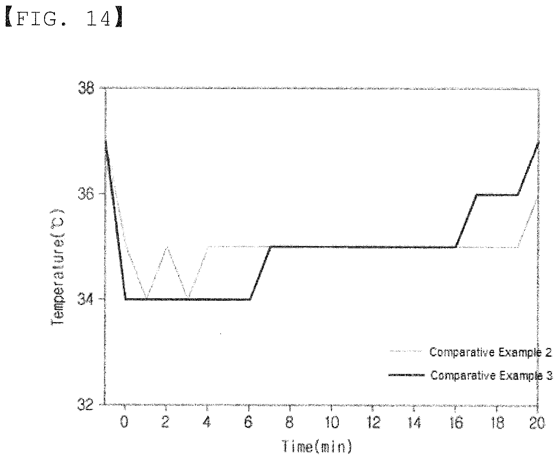

Each of the sunscreen formulation containing menthol prepared according to Comparative Example 3 and the sunscreen formulation of Comparative Example 2 was applied to a forearm area, skin temperature lowering and duration were measured, and the results are shown in the graph of FIG. 14.

As shown in FIG. 14, after applying the sunscreen formulation containing menthol of Comparative Example 3, the skin temperature was lowered by 3°C and the temperature reached the same temperature as the skin after 20 minutes, thereby showing similar results to the sunscreen formulation containing no PCM microcapsule of Comparative Example 2. When the menthol-containing sunscreen was applied, it was confirmed that a refreshing feeling was felt, but there was no cooling effect due to the menthol component.

As such, it was confirmed that when the PCM microcapsule prepared in the Example of the present invention served to have excellent cooling feeling and durability in the sunscreen formulation.

As described above, although the present invention has been described with reference to exemplary embodiments and the accompanying drawings, it would be appreciated by those skilled in the art that the present invention is not limited thereto but various modifications and alterations might be made without departing from the scope defined in the range of equivalents of the following claims.

## Claims

1. A core-shell PCM microcapsule having an automatic temperature control function, comprising: an inner core of a PCM and an outer shell of a biodegradable polymer.

2. The core-shell PCM microcapsule having an automatic temperature control function of claim 1, wherein the PCM is included at 5 wt% to 80 wt% with respect to a total weight of the microcapsule composition.

3. The core-shell PCM microcapsule having an automatic temperature control function of claim 1, wherein as the PCM, n-octacosane, n-heptacosane, n-hexacosane, n-pentacosane, n-tetracosane, n-tricosane, n-docosane, n-heneicosane, n-eicosane, n-nonadecane, n-octadecane, n-heptadecane, hexadecane, n-pentadecane, n-tetradecane, n-tridecane, stearic acid, and derivatives or composites thereof are used alone or in combination to adjust a melting point of the PCM.

4. The core-shell PCM microcapsule having an automatic temperature control function of claim 3, wherein the PCM is n-octadecane and n-eicosane having a melting point of 28-35°C for a cooling cosmetic, and n-octadecane and n-eicosane are used alone or n-docosane having a melting point of 44°C is mixed with n-octadecane at a constant ratio.

5. The core-shell PCM microcapsule having an automatic temperature control function of claim 1, wherein as the biodegradable polymer, polycaprolactone, polylactic acid, water-insoluble cellulose, polyhydroxybutyrate, and aliphatic-based unsaturated polyester compounds are used alone or in combination.

6. The core-shell PCM microcapsule having an automatic temperature control function of claim 1, further comprising a functional substance at a weight ratio of 1 to 50 with respect to a weight of the PCM in the capsule.

7. The core-shell PCM microcapsule having an automatic temperature control function of claim 6, wherein the functional substance is any one of menthol, a natural oil, a synthetic oil, an essential oil, a fragrance, a vitamin, a ceramide, and an organic UV absorber.

8. The core-shell PCM microcapsule having an automatic temperature control function of claim 1, wherein the core-shell PCM microcapsule has an average particle size of 0.05 µm to 50 µm.

9. A method of preparing a core-shell PCM microcapsule having an automatic temperature control function, the method comprising:
dissolving a shell material which is a biodegradable polymer and a PCM which is a core material in a solvent to produce an oil phase polymer solution,
dissolving an aqueous polymer to produce an external continuous phase,
emulsifying the oil phase polymer solution and the external continuous phase, and
removing the external continuous phase and the solvent and carrying out drying.

10. The method of preparing a core-shell PCM microcapsule having an automatic temperature control function of claim 9, wherein the PCM is included at 5 wt% to 80 wt% with respect to a total weight of the microcapsule composition.

11. The method of preparing a core-shell PCM microcapsule having an automatic temperature control function of claim 9, wherein as the PCM, n-octacosane, n-heptacosane, n-hexacosane, n-pentacosane, n-tetracosane, n-tricosane, n-docosane, n-heneicosane, n-eicosane, n-nonadecane, n-octadecane, n-heptadecane, hexadecane, n-pentadecane, n-tetradecane, n-tridecane, stearic acid, and derivatives or composites thereof are used alone or in combination to adjust a melting point of the PCM.

12. The method of preparing a core-shell PCM microcapsule having an automatic temperature control function of claim 9, wherein as the biodegradable polymer, polycaprolactone, polylactic acid, water-insoluble cellulose, polyhydroxybutyrate, and aliphatic-based unsaturated polyester compounds are used alone or in combination.

13. The method of preparing a core-shell PCM microcapsule having an automatic temperature control function of claim 9, wherein the core-shell PCM microcapsule has an average particle size of 0.05 µm to 50 µm.

14. The core-shell PCM microcapsule having an automatic temperature control function of claim 9, wherein a functional substance is further included at a weight ratio of 1 to 50 with respect to a weight of the PCM in the capsule.

15. The core-shell PCM microcapsule having an automatic temperature control function of claim 9, wherein the functional substance is any one of menthol, a natural oil, a synthetic oil, an essential oil, a fragrance, a vitamin, a ceramide, and an organic UV absorber.

16. A cosmetic composition for external skin comprising 0.1 wt% to 30 wt% of the core-shell PCM microcapsule of any one of claims 1 to 8 with respect to a total weight of the cosmetic composition.
